Europäisches Patentamt

European Patent Office

Office européen des brevets

(11) Publication number: **0 305 561**
**A1**

## EUROPEAN PATENT APPLICATION

(21) Application number: 87112707.2

(22) Date of filing: 01.09.87

(51) Int. Cl.⁴: **A62D 1/00 , C11D 1/86 , C11D 3/28**

The title of the invention has been amended (Guidelines for Examination in the EPO, A-III, 7.3).

(43) Date of publication of application:
**08.03.89 Bulletin 89/10**

(84) Designated Contracting States:
**AT BE CH DE ES FR GB GR IT LI LU NL SE**

(71) Applicant: **Environmental Security Incorporated**
**352 Abbeyville Road**
**Lancaster Pennsylvania 17603(US)**

(72) Inventor: **Greene, Jay S.**
**76 North Grant Street**
**Manheim Pennsylvania(US)**

(74) Representative: **UEXKÜLL & STOLBERG**
**Patentanwälte**
**Beselerstrasse 4**
**D-2000 Hamburg 52(DE)**

(54) **Liquid formulations for use as fire-fighting agents, oil dispersants, household or industrial cleaners, or deodorants.**

(57) A liquid composition includes as a major component thereof a detergent mixture of linear alkylbenzene sulfonate, non-ionic detergent and lauric superamide. Depending upon the particular use of the composition, it also includes effective amounts of other components. In use for fighting Class D (burning metal) fires, the composition includes vitamin B-6, sodium chloride, and bicarbonate of soda and the amount of water is minimized so that it is only enough to properly mix the other components together before they are added to the detergent mixture. When used for fighting Class A and B fires, or for extinguishing (or preventing) burning coal fires, the composition includes vitamin B-6, bicarbonate, and water. When used as an oil dispersant, the liquid composition includes vitamin B-6, bicarbonate, water, and lemon or lime juice. Other formulations are provided when used as an industrial or household cleaner, or deodorizer. The four components making up the detergent mixture are varied in composition too, depending on the designed use.

EP 0 305 561 A1

**MULTI-PURPOSE FORMULATIONS**

BACKGROUND AND SUMMARY OF THE INVENTION

U.S. Patent 4,248,733 discloses a liquid composition that is very useful for cleaning up oil spills or the like, and extinguishing petroleum fires. While the materials and methods disclosed therein have recognized utility, they are not as effective as desired (or in some cases at all) in extinguishing other types of fires besides petroleum fires, or in dispersing petroleum liquid as quickly as desired.

The compositions and methods according to the present invention have been developed from the basic composition disclosed in U.S. Patent 4,248,733 with a view toward greatly enhancing the diversity and the effectiveness of the material in the 4,248,733 patent.

One form of the liquid composition according to the present invention is an excellent Class A and B firefighting agent. It is capable of dilution with water at conventional dilution rates commonly employed by most firefighting equipment. It causes the extinguishing stream to be heavier and, this prevents premature breakup of the stream when very hot fires are being fought. For solid material fires, it causes water to penetrate the natural fiber faster and reach deep-seated fires more efficiently, thereby reducing the amount of liquid per unit time required to extinguish the fire. In liquid fuel fires, it cuts off the oxygen to the fuel thereby breaking the fire chain reaction and cooling the surface by absorbing heat, and changing the surface of the material so as to break down the hydrocarbon chain making the molecules on the surface essentially non-combustible. It prevents re-ignition in most circumstances, diminishes and breaks up fire gases when vapor comes into contact with smoke, and helps prevent spalling and cracking due to absorption into block, brick, concrete slabs, and like porous material. It is utilizable with all types of existing equipment, stationary and mobile, including eductor systems, diaphragm system, sprinkler systems, hand-held hoses and extinguishers, and the like.

Another form of the liquid composition according to the present invention is useful in concentrated form for fighting Class D (burning metal) fires, and in diluted form for fighting Class B fires. It is the only known liquid firefighting agent that can successfully extinguish Class D fires. It is capable of absorbing a tremendous heat production from combustible metals without turning into gas and separating as other liquid agents do when they come in contact with burning combustible metals. Its heat absorption ability is approximately 7.7 million BTUs per pound, compared to 1,142 BTUs per pound for water. It may be applied with any standard mobile or fixed firefighting equipment utilizing a wide angle fog nozzle, or sprinkler system head. When used for extinguishing Class B fires, it is mixed with water at a rate of about 0.5-2% (e.g., 1.5%) and sprayed, onto the fire preferably in a fog pattern at a pressure of about 50-200 pounds per square inch. Extinguishing time is typically about 15-70 seconds, and it greatly minimizes the chance of re-ignition, and requires a low effective application rate.

Another form of liquid composition according to the present invention is suitable for effectively extinguishing burning coal fires, including coal mine fires. It is the only known agent capable of effectively extinguishing coal mine fires. When sprayed onto existing fires at a dilution rate of about 6-10% with water, it emulsifies the coal as well as extinguishing it, preventing re-ignition for extended periods of time. It also attacks mine fire gases and causes less smoke in the fire area. The extinguishing stream is heavier than plain water and thereby holds its properties longer and increases the separation time over plain water. This has the effect of allowing the solution to get to the fire. Also, its heat absorption rate is substantially greater (e.g., 11-20 times as great as that of water) so that it effects faster extinguishment and with emulsification changes the combustible matter into a, much less combustible material, which in turn breaks the chemical chain reaction and it extinguishes the fire. It also will extinguish methane gas fires on contact, although it will not prevent re-ignition of the methane gas fire unless the source of the gas has been emulsified.

Yet another form of the liquid composition according to the present invention is ideally suited for dispersing petroleum liquids polluting environmental systems, such as salt water, fresh water, or land. Generally, one application of the agent mixed with water will cause the dispersal and elimination of 4% of the polluting petroleum liquid per 24 hours after application is complete. Continued reaction can be expected for up to about a week without reapplication, and application can be repeated. The composition generally operates first as an emulsifier causing the density of the oil or other petroleum product to decrease very dramatically, and then starts to demulsify. In water this causes the oil to surface at a lower weight density. Both emulsification and demulsification continue until the oil is totally eliminated as a hydrocarbon. On land, the agent may be used to dislodge heavy oil buildup with the effect of making clean up with a vacuum system more effective and quicker. It also can be used to clean oil soaked birds and animals without harming them. For water spills, liquid composition is typically metered at a dilution rate of about 10-25% with water, and is spread as evenly as possible. Heavy initial application is avoided, and reapplication can be provided as necessary. On land, it is metered at about a rate of 25% to water, and for cleaning birds and animals is metered to water at a rate not to exceed 6%. It also will extinguish a fire on an oil slick, but should be used only with fires involving heavy oils. Any existing fire pumping equipment can be utilized to apply a dispersant, as may most hoses or pipe systems now in use to dispense metered amounts of liquids.

Also according to the present invention a particular industrial cleaner is provided with a low pH (i.e., between about 7.6-8) which provides easy grease removal and other cleaning action, will not cause harm to the environment, is safe to use and store without warning labels, requires no special handling, and is non-combustible in excess of 2000° F.

Also according to the present invention a commercial deodorant is provided which may be used as a concentrate, or mixed with water, and used in essentially any environment containing undesirable odors. The deodorant is non-toxic, biodegradable, has a low pH, is non-flammable and noncombustible, will not cause harm to the environment, and may be used as a solid, liquid or particlized spray.

Each formulation of the invention has as a major component thereof a household liquid detergent, comprising a linear alkylbenzene sulfonate, non-ionic detergent and lauric superamide detergent mixture. A typical such detergent mixture is the LC product manufactured by Best Lane Products, Inc. While thah particular detergent mixture is effective it has been found that by varying components and proportions of components of the detergent mixture depending upon the ultimate use for the detergent mixture, the final properties of the formulations produced may be enhanced.

The basic components of the detergent mixture are linear alkybenzene sulfonate (LAS) isoocytylphenyl polyethoxyethanol (IP), polyoxyethylene sorbitan monooleate (PSM), lauric diethanolamide (LD), monoethanolamide superamide (MS), and water. LAS, LD, and MS are solids that must be brought to a liquid with heat, and IP and PSM are then added hot or cold, depending upon the desired effect. The temperature range for making the prime chemical is from about 125-200° F and the differences in temperature also can change the characteristics of the chemical. Thus, pH, viscosity, surface tension, and specific gravity can be changed at will depending upon the particular proportion of the components of the detergent mixture, and are made slightly different for each formulation. For instance, different components and proportions of components of the detergent mixture are provided depending upon whether the final formulation is to be used as a class A fire emulsifier, a class A and B fire emulsifier and foaming agent, a class B and D firefighting agent, a class D fire fighting agent, an oil dispersant, a coal and tire fire fighting agent, a heavy industrial cleaner, a light cleaner, or a commercial deodorant.

## DETAILED DESCRIPTION OF THE INVENTION

The liquid formulation according to the present invention comprises a detergent mixture as described above. An effective amount of other materials are also added to the detergent mixture depending upon the particular use, and the components of the detergent mixture are varied depending upon end use. All materials employed in the liquid composition according to the invention are non-toxic so that they may be used in essentially any environmental system.

The liquid formulation of the invention includes vitamin B-6. The vitamin B-6 acts as a densifier, and for firefighting application increases the heat absorption capability of the detergent mixture.

For all the firefighting applications of the liquid composition according to the invention, alfalfa is an optional element that may be utilized as a component of the formulation. If used, the alfalfa slows down the emulsification rate of the detergent mixture so that the detergent suds stay on the surface longer. The alfalfa also has a minor densifying action (that is, it intensifies the sudsy surface a small amount).

For all the uses of the liquid composition according to the invention extinguishing use, bicarbonate of soda is also a significant component of the formulation. The bicarbonate of soda agitates the detergent mixture so that it suds more quickly, and acts as a stabilizer. In general, it helps prevent burn-through in firefighting applications.

For the Class B and D firefighting applications, particularly the Class D firefighting applications, a significant component of the liquid formulation comprises sodium chloride. Sodium chloride densifies the detergent mixture, and prevents it from separating and detonating in Class D fires. It partially dissolves in the detergent mixture down to the molecular level, and will not itself cause detonation. Other commonly-recognized Class D agents are not effective, nor are other salts such as potassium chloride (e.g., potassium will burn when exposed to a burning combustible metal). For the oil dispersing uses, lemon or lime juice is added to the liquid formulation. Lemon or lime juice is added since they are the only known non-toxic (and environmentally sound) defoamers. They speed up the emulsification reaction (and thus in the oil dispersing applications no alfalfa should be used since it slows down the emulsification reaction).

Water is also a significant component of each of the liquid formulations except for the Class D firefighting composition. The water is provided to increase cost effectiveness, and to reduce the weight of the final product. Also, the water is added so as to provide adjustment of the volumetric quantity of the liquid composition so that it can be applied at conventional dilution rates utilizing conventional equipment. For the Class D firefighting agent, the amount of water is minimized since water will cause the agent to be less effective and may cause separation or detonation. Only enough water is added, to the components, besides the detergent mixture, so that they can be mixed properly prior to addition to the detergent mixture during production of the liquid formulation.

In all cases, non-toxic and (in the case of the firefighting applications) low flammability, coloring and perfuming agents are added so that the liquid formulation does not have an objectionable appearance or odor. The most suitable coloring material is standard vegetable color (such as blue vegetable color for the oil dispersing material), and the best known perfuming agent is eucalyphus oil. These materials are typically added so that their combined total contribution to the liquid formulation is less than about 4% by weight of the detergent mixture.

For the Class A and B firefighting agent, bovine urine is an optional component thereof. When utilized, the bovine urine provides uric acid which helps in the sudsing action of the detergent mixture, which sudsing action prevents oxygen from getting to the combustible material.

A cleaner for industrial uses comprises about 45-75% by volume detergent mixture, vitamin B-6 in an amount of about 0.5-3% by weight of the detergent mixture, vegetable color (when used) in an amount of about 0.5-2% by weight of the detergent mixture, and water comprising 25-55% by volume of the cleaner. The cleaning agent has hundreds of uses, including as a tank cleaner, a bilge cleaner, a cleaner for animal houses, including cesspits, and for neutralizing common acids. The cleaning agent is water activated and is mixed with varying amounts of water depending upon particular end uses to which it is put.

The commercial deodorant has as basic components a detergent mixture, vitamin B-6, vegetable color (optional), sodium bicarbonate, fragrance and water. The sodium bicarbonate along with the detergent mixture (particularly the linear alkybenzene sulfonate components thereof) produces sufficient viscosity of the formulation so that the fragrance is properly held. Even when the concentrated deodorizer is diluted, the mixture remains intact and will not separate. In higher concentrations it may be a semisolid that can be remixed and cut to a desired consistency and in lower concentration, it is immediately available for use either as a concentrate, or diluted with water, or as an additive to conventional cleaners.

General characteristics and functions of the various forms of the liquid composition of the present invention having been described, details of the compositions thereof and methods of utilization thereof will now be described. In the following description, except where indicated, the expression "by weight" is expressed as a percentage of the weight of the detergent mixture of alkylbenzene sulfonate, nonionic detergent and lauric superamide. For example, if the bicarbonate contribution to the liquid composition is indicated as 0.25-20% by weight, this means that the amount of bicarbonate is from 0.25% of the weight of the detergent mixture component of the composition, to 20% by weight of the detergent mixture contribution to the composition.

In the following descriptions also, the liquid formulation may comprise, or be limited to, the indicated components.

## Class A and B Firefighting Agent

This liquid composition will typically be applied at a dilution of rate of about 3-6%, and the listing of the quantities of ingredients herein is made with such a dilution rate in mind. Of course, for other dilution rates, the same agent components can be utilized, with adjustments in the quantities and/or percentages of each corresponding to the other desired dilution rates. The agent may be used with all types of conventional firefighting equipment, and is preferably applied at any pressure from about 20 pounds per square inch - 400 pounds per square inch. The agent can totally extinguish essentially all Class A and B fires in 9-90 seconds, including natural wood, plywood, particle board, plastic building products, roofing materials, crude oil, motor oil, jet fuel, fuel oil, kerosene, diesel oil, and gasoline fires.

This liquid composition preferably comprises the following composition of ingredients:

Detergent mixture of linear alkylbenzene sulfonate, nonionic detergent and lauric superamide, about 22-45% by volume of the total liquid composition;

Alfalfa - 0-10% by weight;

Vitamin B-6, 0.5-1.5% by weight;

Bicarbonate of soda - about 0.25-20% by weight;

Bovine urine - 0-10% by weight; and

Water, about 50% by volume (variable depending upon specific dilution rate).

Preferably alfalfa and bovine urine are not used at all.

Also, the color and odor of the liquid composition are usually desirably adjusted, as by providing 0.25-1% by weight vegetable color, and 1-2% by weight eucalyptus oil.

When the agent is to be utilized as a class A fire emulsifier (not a foaming agent), the detergent mixture ideally comprises the following components based on volume measurement: about 10-30% LAS, about 8-12% IP, about 1-5% LD, about 0.25-0.9% MS, and 52.1-80.75% water.

When the fire fighting agent is to be utilized as both a class A and B fire emulsifier and foaming agent for flammable and combustible liquids as well as structural fire fighting, the detergent mixture preferably comprises (by volume): about 20-35% LAS, about 7-10% IP, about 3-12% PSM, about 4-10% LD, about 0.5-1.5% MS, and about 31.5-65.5% water.


EXAMPLE I

A liquid formulation of Class A and B firefighting agent was produced by mixing all of the components except for the detergent mixture and vegetable color with water, and then pouring the detergent and vegetable color over the mixture while stirring. After mixing is complete, the formulation included, per 4.1 pounds of detergent mixture, 1% bovine urine, 0.25% vegetable color, 1.8% eucalyptus oil, 3% alfalfa, 1% vitamin B-6, and 12% bicarbonate of soda, with about one-half of the total volume of liquid composition being water. This liquid composition was used to extinguish a number of different types of Class A and B fires, with the results indicated in Tables I and II:

5

## TABLE I

| Test Number | 1 | 2 | 3 | 4 | 5 | 6 |
|---|---|---|---|---|---|---|
| Depth, Fuel-Before | 2" | 2" | 2" | 2.5" | 12C/F | 19.6C/F |
| Depth, Fuel-After | 1-7/8" | 1-15/16" | 1-15/16" | 2-7/16" | 9.25C/F | 18C/F |
| Total Sq. Footage | 10 | 10 | 10 | 10 | 3.75C/F | 1.64 C/F |
| Total BTU Output | 901,440 | 901,440 | 901,440 | 901,440 | 5,030,662 | 29,520 |
| Total So. Used | 63 oz | 41 oz | 42 oz | 25 oz | 256 oz | 236 oz |
| % Concentration of Agent | 6 | 3 | 2 | 1 | 10 | 6 |
| % Sol. Per Sq.Ft. | 6.3 oz | 4.1 oz | 4.2 oz | 2.5 oz | 75 oz | 12 oz |
| % Con. Per Sq.Ft. | .378 oz | .123 oz | .084 oz | .025 oz | 6.9 oz | .722 oz |
| Total Con. Used | 3.78 oz | 1.23 oz | .84 oz | .25 oz | 26 oz | 14.16 oz |
| Gallons Per Min. | 1.9 | 1.9 | 1.9 | 1.9 | 1.9 | 1.9 |
| Extinguishing Sys. | W/P | W/P | W/P | W/P | W/P | W/P |
| Nozzle Pressure | 1.1 | 1.1 | 1.1 | 1.1 | 1.1 | 1.1 |
| Velocity of Dis. | 12.7 | 12.7 | 12.7 | 12.7 | 12.7 | 12.7 |
| Air Temperature (°F) | 48 | 48 | 53 | 53 | 48 | 57 |
| Type of Fuel | Oil#6 | Oil#6 | Oil#6 | Oil#6 | Coal/A | Tires |
| Time of Ignition | 1141 | 1221 | 1259.31 | 1327.40 | 0920 | 1410 |
| Dur. of Ext-Start | 1143 | 1222.11 | 1300.50 | 1328.12 | 1425 | 1413 |
| Dur.of Ext-Finish | 1143.16 | 1222.24 | 1301.03 | 1328.22 | 1430 | 1413.16 |
| Time to Extinguish (in minutes) | 0.16 | 0.13 | 0.13 | 0.10 | 5 | 0.36 |
| Density Per Sq.Ft. | .043 | .028 | .029 | .017 | 2.0 | 1.84 |

## TABLE II

| Test Number | 7 | 8 | 9 | 10 | 11 |
|---|---|---|---|---|---|
| Depth, Fuel-Before | 2" | 4' | 4" | 2" | 3" |
| Depth, Fuel-After | 1-15/16" | 3-7/8" | 3-7/8" | .5" | 2-1/2" |
| Total Sq. Footage | 10 | 5.25 | 5.25 | 5.25 | 5.25 |
| Total BTU Output | 457,700 | 624,000 | 624,000 | 752,000 | 640,000 |
| Total, Sol. Used | 128 oz | 96 oz | 56 oz | 102 oz | 44 oz |
| % Concentration | 3 | 100 | 3 | 3 | 3 |
| % Sol. Per Sq.Ft. | 12.6 | 6 | 10.5 | 19.43 | 8.34 |
| % Con. Per Sq.Ft. | .04 | 6 | .32 | .59 | .25 |
| Total Con.. Used | 4 oz | 32 oz | 1.68 oz | 3.06 | 1.32 |
| Galls Per Min. | 5.45 | 5.45 | 5.45 | 5.45 | 5.45 |
| Extinguishing Sys. | W/P | W/P | W/P | W/P | W/P |
| Nozzle Pressure | 20 | 20 | 20 | 20 | 20 |
| Velocity of Dis. | 56 | 56 | 56 | 56 | 56 |
| Air Temperature (°F) | 70 | 74 | 74 | 77 | 54 |
| Type of Fuel | Diesel | Kerosene | Kerosene | Gas | Diesel |
| Time of Ignition | 1457.11 | 1613 | 1634 | 1345 | 1550 |
| Dur.of Ext-Start | 1458.44 | 1613.32 | 1634.25 | 1345.20 | 1551.32 |
| Dur.of Ext-Finish | 1458.55 | 1613.50 | 1634.45 | 1353 | 1552.13 |
| Time to Extinguish (in minutes) | 0.11 | 0.18 | 0.20 | 7.80 | 0.81 |
| Density Per Sq.Ft. | .1 | .08 | .08 | .18 | .058 |

Class B and D Firefighting Agent

The same basic firefighting agent can be formulated for both Class B and D fires. For Class D fires, however, it is applied at essentially 100% concentration. If water is added, it will decrease effectiveness and may cause separation and detonation. It is applied at a system pressure desirably no less than 50 pounds per square inch, and preferably with the range of about 50-200 pounds per square inch. It is desirably not applied with a straight stream nozzle as the pressure of any stream against the combustible metal tends to cause hot metal fragments to be dispersed in all directions. It is best applied with a wide angle fog nozzle, or existing sprinkler systems which have heads which will break up the liquid stream into a dispersed pattern.

When used for fighting Class B fires, it is mixed with water prior to application to the fire, at a dilution rate of about 0.5-2% of water (e.g., 1.5%). It is preferably applied with a minimum system pressure of 60 pounds per square inch, and preferably is applied with a fog nozzle although a straight stream may be utilized where the firefighting equipment is at a great distance from the fire.

The basic ingredient of the liquid firefighting formulation according to this aspect of the invention is a detergent mixture comprising a linear alkylbenzene sulfonate, non-ionic detergent and lauric superamide detergent mixture. In the preferred embodiment that mixture comprises about 39-67% of the total mass of the formulation. It has been found particularly effective if the detergent comprises, by volume: about 41-45% linear alkylbenzene sulfonate, about 8-12% isooctylphenyl polyethoxyethanol, about 0-4% polyoxyethylene sorbitan monooleate, about 8-12 lauric diethanolamide, about 0.5-1% monoethanolamine superamides, and about 26-30 water.

The formulation according to the invention also preferably comprises vitamin B-6 in an amount of about 1-3%, with about 1% being optimum, by weight of the detergent mixture. The vitamin B-6 acts as a densifier and increases the heat absorption capability of the detergent mixture.

The liquid composition according to the invention also preferably comprises sodium chloride as a significant component thereof. Sodium chloride typically comprises about 3-41% by weight of the detergent mixture, with about 37% being optimum. Sodium chloride densifies the detergent mixture, and prevents it from separating and detonating in Class D fires. It partially dissolves in the detergent mixture down to the molecular level, and will not itself cause detonation. Other commonly-recognized Class D agents are not effective, nor are other salts such as potassium chloride (e.g. potassium will burn when exposed to a burning combustible metal).

Another significant component of the preferred liquid composition according to the invention is bicarbonate of soda. The bicarbonate of soda is typically provided in the amount of about 3-20% by weight of the detergent mixture, with about 8% being optimum. The bicarbonate of soda agitates the detergent mixture so that it suds more quickly, and acts as a stabilizer, and helps prevent burn-through.

Alfalfa at 0-7% can be used, but is not preferred.

The liquid composition according to the invention also requires the use of some water in order to provide effective mixing of the other constituents. However, the amount of water is minimized since water will cause the agent to be less effective and may cause separation or detonation. Only enough water is added to the components, besides the detergent mixture, so that they can be mixed properly prior to addition to the detergent mixture during production of the liquid formulation.

In the method of formulating the Class D liquid firefighting agent according to the invention, effective amounts of vitamin B-6, sodium chloride, and bicarbonate of soda are added to a minimum amount of water sufficient to facilitate mixing of the components. Then the vitamin B-6, sodium chloride, and bicarbonate of soda and water are mixed together so that substantially no lumps exist and a generally homogeneous slurry is provided. Then the slurry is mixed with the detergent mixture so as to provide a homogeneous liquid combustible metals firefighting agent.

The Class D firefighting agent according to the invention is capable of fighting all combustible metal fires. For example it can extinguish titanium, magnesium, zirconium, graphite, and uranium fires. Even if the metals are radioactive (such as uranium, plutonium, or graphite which has become radioactive due to close contact over a long period of time with uranium, plutonium, or the like), the agent is effective to extinguish the fire within a short period of time.

## EXAMPLE II

A Class D firefighting liquid composition was formulated by mixing 8.25 pounds of detergent mixture with other components. One percent by weight eucalyptus oil, 1% by weight vitamin B-6, 37% by weight sodium chloride, and 8% by weight bicarbonate of soda were mixed with a minimum amount of water, only enough to provide effective mixing of the components. The amount of water sufficient to do this would typically be about 3-7% by weight, with mixing taking place until there are no lumps. The detergent mixture and 0.5% by weight vegetable color are then added to the other components, and mixing continues until a homogeneous product is produced. This product is then suitable for direct application to Class D fires by spraying in on the fires with a fog nozzle, or the like. Utilizing this liquid composition as a firefighting agent, the following test was conducted:

## TEST SET UP

Titanium powder (99.8 min) was placed on a 6 square foot steel plate in an 8 foot pit. The depth of the titanium ranged from 2 to 2.5 inches. The test was conducted in the pit to reduce the possibility of agent splashing upon application. Then a small quantity of magnesium was blended with the titanium powder. Next, the powder mixture was saturated with gasoline. The magnesium and gasoline were added to act as accelerants upon ignition of the titanium powder.

Test Procedure

A torch was applied to the powder mixture described in the test set up. The metal mixture was allowed to burn for 7.5 minutes (pre-burn). After 7.5 minutes the subject agent was applied to the metal fire by an Akron turbo-jet nozzle rated at 30 gal/min and a pressure of 100 psig. This first application, consumed 15 gallons of agent. At this point the metal surface fire was not completely covered. An additional 5 gallons of agent was applied approximately 5 minutes later so that the metal was completely engulfed in agent.

Observations

The following observations were made after the second application of agent at the time intervals listed below.

15 minutes - Agent was adhereing to the metal surface. The agent at the center of the pit was bubbling. No signs of fire.

30 minutes - Some bubbling of the agent still visible. No signs of fire.

45 minutes - Test personnel concluded that the fire was completely extinguished, no signs of bubbling or fire. The agent was removed from the test pit, the remaining titanium powder appeared caked. In order to reach the core of the metal the outside surface had to be chipped away. Upon reaching the core, it was observed that the core temperature was less than the ambient temperature of the pit.

It was concluded that the titanium fire was extinguished and the temperature of the titanium was low enough not to warrant re-ignition.

While of course small amounts of contaminants are tolerable, with no adverse affect on the ability of the firefighting agent according to the invention to extinguish combustible metal fires, it is desirable that the agent include only the detergent mixture, vitamin B-6, sodium chloride, bicarbonate of soda, a minimal amount of water, and small amounts of non-toxic and low flammability perfuming and coloring agents.

Fire Extinguishing of Burning Coal

As will be seen in Test Number 5 in Table I above, coal fires are significantly more difficult to put out than common Class B fires. The following liquid formulation, however, can be successfully utilized to. extinguish coal fires, including coal mine fires.

The liquid formulation comprises detergent mixture, vitamin B-6, bicarbonate of soda, water, and non-toxic coloring and perfuming agents if desired. Preferably no other materials are used. More specifically, the formulation preferably comprises a mixture of: the detergent mixture which comprises about 50% by volume of the formulation; vitamin B-6 in the amount of about 0.5-3% by weight of the detergent mixture; bicarbonate of soda in the amount of about 3-18% by weight of the detergent mixture; and water comprising about 37-47% by volume of the total formulation. If a non-toxic coloring agent is utilized, preferably red vegetable color, it is utilized in the amount of about 0.25-0.75% by weight of the detergent mixture. When a non-toxic perfuming agent is utilized, such as eucalyptus oil, it is preferably utilized in the amount of about 1-2% by weight of the detergent mixture.

The formulation according to the invention can be used in coal firefighting applications of all types. For instance it can be used in deep mines by direct manual hose lines, deep mines with fixed sprinkler systems, penetration of currently closed mines by boring holes therein and pumping the agent into the bore holes, and underground fires involving packed or graded areas, and coal piles either binned or open and awaiting use or shipment, in rail or sea operation where spontaneous ignition has occurred or must be prevented, and on tire dump sites. Maximum utility is achieved when the detergent mixture comprises (by volume) about 10-40% LAS, about 2-12% IP, about 0-5% PSM, about 2-10% LD, about 0-2% MS, and about 31-86% water. For most applications, where the formulation is metered to the fire at about 6-10% dilution rate to water, the most desirable proportion of the components are: the detergent mixture comprising (by volume) 33% LAS, 6% IP, 3% PSM, 6% LD, 2% MS, and 50% water (this is referred to hereafter in the specification as "the specific detergent mixture"), the detergent mixture itself comprising 50% by volume of the total formulation; bicarbonate of soda 8%; vitamin B-6 1/2%; red vegetable color about 0.5%; and water comprising the rest of the formulation.

According to the invention, it is highly desirable to use red dye in the formulation. The red dye is used as an indicator to tell the firefighters when an area has been extinguished so that they can move on further down in the coal mine tunnel or shaft. The red dye is provided in an amount effective to cause a red

9

colored light to be reflected when a light source is directed onto the coal surface when the coal to which the agent has been applied is no longer burning. When the firefighters see the red reflected light, they proceed further to other areas. Thus according to the invention a method of fighting a fire is provided which comprises the steps of: (a) Applying directly to the burning coal a liquid composition containing a mixture of: a linear alkylbenzene sulfonate, non-ionic detergent and lauric superamide detergent mixture; vitamin B-6; bicarbonate of soda; water; and red dye so as to put out fire without generation of gases toxic to humans; (b) Directing a light on portions of the coal to which the liquid composition has been applied; (c) Detecting the reflection of the light off of the coal to which the liquid composition has been applied; and (d) Proceeding past the area upon which the light has been directed once there is a reflection of red colored light, provided by the red dye, from that area.

Utilizing the formulation according to the present invention, it is also possible to practice methods of absorbing methane and other gases generated by a fire, and retarding spontaneous ignition of coal.

That is, according to the present invention a method of absorbing gases generated by a coal burning fire, including methane, is provided comprising the step of spraying into the air in the area where the gases are present, metered with water, a formulation comprising a mixture of a linear alkylbenzolyate sulfonate, non-ionic detergent and lauric superamide detergent mixture; vitamin B-6; bicarbonate of soda; and water. This method is practiced by metering the liquid formulation at about a 0.25-0.5% dilution rate to water. The finer the spray, the more quickly the absorbing action will take place. The formulation absorbs $CO_2$, $CO$, $NO_2$, and $SO_2$. This gas absorbing function inherently takes place when the formulation according to the invention is used in firefighting, methane and like gases being absorbed as generated.

According to the invention there is also provided a method of retarding spontaneous ignition of coal comprising the steps of: (a) providing a liquid composition comprising a mixture of linear alkylbenzolyate sulfonate, non-ionic detergent and lauric superamide detergent mixture; vitamin B-6; bicarbonate of soda; and water; and (b) spraying the liquid composition, metered with water, onto the coal surface area at which it is desired to retard spontaneous ignition. For this method, the liquid formulation is metered at about a 0.5-1.5% dilution rate to water. Spontaneous ignition will be prevented indefinitely as long as no significant increase in temperature or pressure results.

When used for firefighting, the liquid composition is applied as a water additive at a rate of application of about 6-10% to water. For application against hot mine surfaces where there is little open flame, a 6% metering rate is sufficient, while for coal faces that have an open flame and high pressure, a 10% metered rate is sufficient. Fire gases will be dispelled in both situations. For tunnel areas with smoke and heat from hot walls etc., and no open flame, 3% metering of agent to water is preferred. For mine entrance areas or just outside mine entrances, 3% metering of agent to water is preferred.

Application is best by a disperse spray of about 9-125 gallons per minute covering the surfaces of the affected areas, although a straight stream may be used in some circumstances. Nozzle pressure should typically be about 50-90 psi. For closed mine operation, the liquid composition is mixed to water at a 6-10% rate, and can be injected through bore holes in the top of the mine with an injection pipe placed down into the mine cavity. A deflector may be installed on the bottom end of the pipe to cause faster break above the stream as it enters the mine cavity.

EXAMPLE III

For 4.1 pounds of the specific detergent mixture described above, 1% by weight eucalyptus oil, 8% by weight bicarbonate of soda, and 0.5% by weight vitamin B-6 were mixed with water. After thorough mixing, the detergent mixture and 0.25% by weight red vegetable color were added, and mixing continued until a homogeneous liquid composition was produced. The liquid composition was sprayed directly on the faces of a mine tunnel in a coal mine that was on fire, and successfully extinguished the fire in the treated area of the mine tunnel.

EXAMPLE IV

Four different tests were conducted to determine the amount of agent that was desirable to use in extinguishing a coal fire, and to compare the results of using the agent according to the invention with the results using water, and water and foam. The "agent" utilized in these tests was the preferred agent described above, in which the detergent mixture comprised 33% LAS, 6% IP, 3% PSM, 6% LD, 2% MS, and 50% water, the detergent mixture itself comprising about 50% by volume of the total formulation and the total formulation also including about 8% by weight of the detergent mixture of bicarbonate of soda, about 0.5% by weight of the total detergent mixture of vitamin B-6, about 0.5% vegetable coloring, and about 1% eucalyptus oil, and the remainder water.

Test Equipment, Test #s 1 and 3:

A 55 gallon drum was split lengthways and the edges turned out. The drum was then turned horizontally and a quarter-inch steel grate placed inside, approximately eight inches from the bottom. One inch of water and six inches of diesel fuel were loaded under the grate. Coal was then placed on the top of the grate.

Test Equipment, Test #s 2 and 4:

A 20 square foot pan with four-inch sides was used. The pan has legs attached and stands four inches off the ground. Each leg is equipped with adjusters so the pan may be leveled. Several steel grates with a 1/4″ space below were placed in the bottom of the pan to provide a good draught for the fire.

Test Procedures, Test #1:

The fuel was ignited with a little gasoline and allowed to burn until the coal pile was fully engulfed. Extinguishment was then recorded with all the data as follows.

Test Procedures, Test #2:

In excess of 1000 lbs. of coal was loaded in the pan on top of the grates. The coal was soaked in 5 gls. of kerosene for four hours prior to ignition. Just before ignition another 5 gls. of kerosene was poured over the coal and one cup of gasoline was used as a starter. The coal pile was ignited and was allowed to burn for 25 hours prior to extinguishment.

Test Procedures, Test #3:

Almost 1200 lbs. of coal were placed in the pan and soaked in five gallons of kerosene. About 20 hours later the pile was ignited with a small amount of gasoline. The fire was checked throughout the night an into the early hours of the next day.

Test Procedures, Test #4:

1200 lbs. of coal was placed in a pan on top of grates. 18 gallons of kerosene was poured into the pan and at 1000 hours with about a gallon of gasoline as a starter fuel the fire was ignited. The coal was allowed to burn for 25 hours to reach the temperature desired before the extinguishment process was started.

### FIRE INFORMATION CHART #1

| Test nr. | 1 | 2 | 3 | 4 |
|---|---|---|---|---|
| 1) Fuel Type | Stove Coal | Anthracite | Anthracite | Anthracite |
| a. Amount | 300 Lbs. | 1200 Lbs. | 1200 Lbs | 1200 Lbs |

| | | | | |
|---|---|---|---|---|
| b. BTU Output per LB | 11,000 | 13,200 | 13,200 | 13,200 |
| c. BTU Output per Hr | 133,650 | 580,000 | 523,072 | 580,000 |
| d. Total BTU Cap. | 2,475,000 | 13,200,000 | 13,200,000 | 13,200,000 |
| 2) Starter Fuel | | | | |
| Type | Diesel | Kerosene | Kerosene | Kerosene |
| a. Amount | 13 Gal | 10 Gal | 5 Gal | 18 Gal |
| b. BTU Output per LB | 22,000 | 23,000 | 23,000 | 23,000 |
| c. Total BTU Output | 286,000 | 230,000 | 115,000 | 2,649,600 |
| 3) Coal Burn Time | 6.40 Hrs | 25 Hrs | 20 Hrs | 25 Hrs |
| 4) Ext. Start Time | 15.35 | 1730 | 1330 | 1120 |
| 5) Ext. Complete | 16.24 | 1900 | 1430 | 1220 |
| 6) Agent Type | Inventive Agent | Inventive Agent | AFFF (Foam) | Water |
| a. % to water | 10 | 6 | 6 | N/A |
| 7) Total Solution | 8.325 Gal | 30 Gal | 30 Gal | 30 Gal |
| a. Total agent used | .833 Gal | 1.8 Gal | 1.8 Gal | N/A |
| 8) Start Temperature | 1150-F | 1450-F | 1425-F | 1450-F |
| a. End Temperature | 150-F | 116-F | 750-F | 1200-F |
| 9) Agent Tank Size | 17 Gal | 17 Gal | 17 Gal | 17 Gal |

| | | | | |
|---|---|---|---|---|
| a. Discharge System | Air | Air | Air | Air |
| b. Hose Size | 1" | 1" | 1" | 1" |
| c. Nozzle Size | 3/4" | 1/4" | Nat. Foam 2 GPM | 1/4" |
| d. Nozzle Type | Straight Fog | Dispersed | Air Aspirated | Dispersed |
| 10) GPM | 2.7 | 2.9 | 2 | 2.9 |
| a. PSI | 10 | 5 | 100 | 5 |

THERMAL CONDUCTIVITY CHART #2:

| Test Nr. | 1 | 2 | 3 | 4 |
|---|---|---|---|---|
| Total BTU Capacity | 2,475,000 | 13,200,000 | 13,200,000 | 13,200,000 |
| BTU Hourly Emission Rate | 133,650 | 580,000 | 523,072 | 580,000 |
| Solution Heat Absorption Per Gl. Average | 14,272 | 19,333 | 9,590 | 8,333 |
| Den. Solution per lb. Coal | .236 | .255 | .450 | .569 |
| Density Per Cu/Ft | 22.9 | 24.74 | 43.65 | 55.19 |
| Metered Rate | 10% | 6% | 6% | N/A |
| Maximum, Agent BTU Absorption Cap. per lb. | 20,100 | 12,060 | 1,184 | 1,184 |
| Minimum, Agent BTU Absorption Cap per lb. | 245 | 235 | 180 | 180 |

## Total Heat Capacity Technical Data, Test #1*4

| LBS 6% Inventive Agent | BTU Absorbed | BTU Remaining | Deg. F. Remaining | Deg. F. Reduction |
|---|---|---|---|---|

13

| | | | | |
|---|---|---|---|---|
| | 40 | 482,400 | 1,517,600 | 758 | 242 |
| | 40 | 482,400 | 1,035,200 | 518 | 240 |
| | 20 | 241,200 | 794,000 | 397 | 121 |
| 1* | 20 | 31,066 | 762,934 | 381 | 16 |
| | 20 | 30,368 | 732,566 | 366 | 15 |
| 2* | 20 | 29,160 | 703,406 | 351 | 15 |
| | 40 | 56,096 | 647,310 | 323 | 28 |
| | 40 | 53,345 | 593,965 | 296 | 27 |
| | 40 | 51,030 | 542,935 | 271 | 25 |
| | 40 | 49,124 | 493,811 | 246 | 25 |
| | 40 | 47,497 | 446,314 | 223 | 23 |
| 3* | 20 | 23,272 | 423,041 | 211 | 12 |
| | 20 | 3,600 | 419,941 | 209 | 2 |

1* -- In this particular fire calculation chart, the temperature has fallen just below the optimum boiling point of both the solute and water, so efficiency is reduced dramatically.

2* -- At this point in the chart the liquid capacity of the solute is below maximum specific heat.

3* -- At this point on the chart only the water in the solution will come to a near boil, the solute has lost almost all its specific heat.

NOTE: The remaining BTUs listed for this size fire does not present a heat-up process as the temperature of the pile is well below auto-ignition temperature.

4* -- A simulated fire to set up the math calculations.

Total Heat Capacity Technical Data on Test Fire #2

The fire test chart shows the average BTU/per hr. emissions rate based on the Stephan-Boltsmann constant, but does not show the total heat capacity of the fire. The total BTUs produced by the fire per hour includes surface emissions rate plus internal production or chemical reaction.

In the case of this fire of 1000 lbs. of coal at 1,450°F it was 1,450,000 BTUs per hour. In the case of this fire due to the uniformity of the coal pile and its configuration it is easy to accommodate the calculations created by the extinguishment process with water and 6% inventive agent.

| LBS of Water & Inventive Agent at 6% | BTU Absorbed | BTU Remaining | Deg. F. Remaining | Deg. F. Reduced |
|---|---|---|---|---|
| 25.5 | 301,500 | 1,148,500 | 1,148 | 302 |
| 25.5 | 301,500 | 847,000 | 847 | 301 |
| 25.5 | 301,500 | 545,000 | 545 | 302 |
| 1* 25.5 | 135,000 | 410,000 | 410 | 135 |
| 25.5 | 29,756 | 380,243 | 380 | 30 |
| 25.5 | 29,691 | 350,551 | 350 | 30 |
| 25.5 | 29,625 | 320,925 | 320 | 30 |
| 25.5 | 29,560 | 291,364 | 291 | 29 |
| 25.5 | 29,447 | 261,866 | 261 | 30 |
| 2* 25.5 | 29,401 | 232,434 | 232 | 29 |

255 lbs.
30 Gal. of Solution

2* -- Actual temperature recorded by the thermocouple was 163-F. The variation in the calculations from the initial 25.5 or 3 gal. per minute to the final application was 69-F. The calculations listed above are within 95% of the actual recorded temperature.

1* -- The point at which inventive agent with water loses maximum efficiency is the temperature at which the chemical is at its optimum boiling point.

## Total Heat Capacity Technical Data on Test Fire #3

| LBS of Water & Foam At 6% | BTU Absorbed | BTU Remaining | Deg. F. Remaining | Deg. F. Reduction |
|---|---|---|---|---|
| 16.7 | 19,205 | 1,405,795 | 1,405 | 20 |
| 16.7 | 19,205 | 1,386,590 | 1,386 | 19 |
| 16.7 | 19,205 | 1,367,385 | 1,367 | 19 |
| 16.7 | 19,205 | 1,348,180 | 1,348 | 19 |
| 16.7 | 19,205 | 1,328,975 | 1,328 | 20 |
| 16.7 | 19,205 | 1,309,770 | 1,309 | 19 |
| 16.7 | 19,205 | 1,290,565 | 1,290 | 19 |
| 1* 16.7 | 19,205 | 1,271,360 | 1,271 | 19 |
| 16.7 | 19,205 | 1,252,155 | 1,252 | 19 |
| 16.7 | 19,205 | 1,232,950 | 1,232 | 20 |
| 16.7 | 19,205 | 1,213,745 | 1,213 | 19 |
| 16.7 | 19,205 | 1,194,540 | 1,194 | 19 |
| 16.7 | 19,205 | 1,175,335 | 1,175 | 19 |

15

| | | | | |
|---|---|---|---|---|
| 16.7 | 19,205 | 1,156,130 | 1,156 | 19 |
| 2* 16.7 | 19,205 | 1,136,925 | 1,136 | 20 |

1* -- At the 16 gallon mark, foam and water together shows a temperature of 1,271-F. on the chart. The actual temperature as recorded by the thermocouple was 650-C., or 1,202-F., meaning that at the upper temperature the foam was responsible for a 69-F. drop over the chart.

2* -- At the end of discharge the actual recorded temperature was 750-F. The chart shows the temperature to be 1,136-F., a 386- difference. The foam was responsible for the additional drop in temperature. This infers that foam and water have a slightly higher specific heat than water alone. The chart also indicates that foam has very little capacity to reduce heat at much above 1000-F. and only minimal ability to absorb heat at or below 1000-F.

NOTE: Because 750-F. is above the auto-ignition point of coal, an additional 15 gal. of water with 10% inventive agent was discharged on the fire in 30 seconds. One minute later the temperature of the fire was at 175-F. to extinguish the fire completely.

### COMPARISON OF PROPERTIES OF WATER AND INVENTIVE AGENT

| Agent | Water | Inventive |
|---|---|---|
| Density (lbs/gl.) | 8.35 | 8.50 |
| Specific Gravity (gr/cm) | 1.00 | 1.05 |
| Viscosity (cps) at 60-F. | 1.00 | 250 |
| Heat of Formation (BTU/lb.) | 180 | 235 |
| Specific Heat of Solution (BTU/lb.) | 1.00 | 2.18 |
| Solute Boil Temp (-F.) | 212 | 425 |
| Heat of Fusion (BTU/lb.) | 80 | 174 |
| Latent Heat of Vaporization (BTU/lb.) | 970 | 231,000 |

### Oil Dispersant

Another form of the liquid composition according to the invention is useful in dispersing petroleum liquid spills, whether in salt or fresh water or on land. For water application, the first application typically is at metered rate to water of about 25% (e.g., about 10-25%), and it is sprayed over the petroleum liquid as evenly as possible. After a 24-hour delay, reapplication can be made at a metered rate to water of about 10%. For land use, the liquid composition is applied at a metered rate of about 25% to water, and after about an hour or several hours, vacuum operations can be begun. The application at the same rate is made as necessary to complete clean-up.

For cleaning birds and animals, the liquid composition is metered to water at rate not to exceed 6%. All water fowl must be thoroughly rinsed off with clean water after treatment. Application can be made with any standard firefighting equipment, sludge and bilge pumps, and the like.

A typical liquid composition of the oil dispersant is
detergent mixture, 50% by volume of the total composition;
vitamin B-6, about 3-10% of the total mixture, by weight;

bicarbonate of soda, about 1-7% of the total mixture by weight;

lemon or lime juice, about 1-3% of the total mixture by weight;

water, about 26-44% by volume of the total mixture; and

where present, vegetable color (e.,g., blue) is 0.1-1% by weight of the total mixture, and eucalyptus oil is about 1-3% by weight of the total mixture. For this formulation, optimum results are achieved when the detergent mixture comprises (by volume): about 25-40% LAS, about 3-8% IP, about 0-3% PSM, about 3-8% LD, about 0-1% MS and 40-69% water.

## EXAMPLE V

4.1 pounds of detergent mixture and 0.25% by weight blue vegetable dye were combined with 1% lemon juice, 7% bicarbonate of soda, 3.5% vitamin B-6, and 1% eucalyptus oil. The eucalyptus oil, vitamin B-6, bicarbonate of soda, and lemon juice were mixed with water, and after thorough mixing, the detergent mixture and vegetable color were added. The resulting liquid composition was applied to an oil spill, and successfully effected dispersing of the oil after initial application, and several reapplications.

### Cleaners

According to the present invention a heavy industrial cleaner can also be produced utilizing as a basic constituent thereof a linear alkylbenzene sulfonate, non-ionic detergent and lauric superamide detergent mixture. The cleaner has a low pH (e.g. about 7.6-8), and may be used for the removal of hydrocarbon matter, grease and other contaminants typically found in an industrial facilities and farm building environments. The cleaning agent is safe, non-combustible, environmentally sound and also may be used as a drain opener. It may be utilized in heavy concentrations, or diluted substantially.

The cleaner according to the invention includes about 45-75% by volume of detergent mixture, vegetable color (when utilized) in an amount of about 0.5-2% by weight of the detergent mixture, vitamin B-6 in an amount of about 0.5-3% by weight of the detergent mixture, and water in an amount of about 25-55% by volume of the total cleaner formulation.

When the cleaner is used for heavy industrial uses and the like, the optimum composition of the detergent mixture is (by volume): about 25-33% LAS, about 5-15% IP, about 0-3% PSM, about 8-10% LD, about 0.5-1% MS, and about 38-61.5% water. This heavy cleaner has hundreds of uses, including the following:

- Fuel and oil tank cleaner, either pressurized or non-pressurized.

- Ship bilge cleaner, pressurized and non-pressurized.

- Oil and grease removal from any metal object.

- Oil and grease emulsifier, when required to be used in an area where standing oil and grease is spilled.

- Industrial cleaner in all areas where hydrocarbons are used as well as dirt removal in an industrial environment.

- Drain cleaner in animal houses.

- Cleaner for all areas of animal houses, including cesspits.

- Cesspits emulsifier to maintain in a semi-liquid state so it may be pumped out without clogging pumps and hose may be used with commercial pumping equipment for animal houses.

The non-ionic bonded solution of the industrial cleaner according to the invention is the "prime mover" in dislodging oil, grease and dirt. Additives to the non-ionic bonded solution should enhance this chemical dislodging operation, as well as helping to maintain it at a low pH and to increase the heat absorption capacity, where desired. Vitamin B-6 is the preferred ingredient for enhancing chemical operation, maintaining a low pH, and increasing heat absorption capacity.

The industrial cleaner according to the invention is water activated and it will be mixed with different percentages of water depending upon the use to which it is put. In the following list the preferred percentage range of cleaner to water is given for the indicated uses:

- with a high pressure system for tank and bilge operations -- 3-10%.

- heavy grease, which is almost in a 25% solid state -- 15-30%.

- light oils and fuels -- 1-5%.

- general cleaning, dirt and dirt mixed with light hydrocarbons -- 3-15%,

- farm drains -- 3-6%.

- pressurized operations for farm buildings -- 1-3%.

17

- farm cesspits -- 15-50%.

While the above percentages of cleaner to water provide good performance without damage to the environment, the percents of components in every cleaner can be increased where desired to perform a special cleaning operation or if increased speed of operation is necessary.

The industrial cleaner according to the invention also may be used to neutralize all common acids, such as sulfuric, hydrochloric, nitric, phosphoric, nitrous, hydrofluoric, perchloric, acetic, carbonic, hydrocyanic and boric acids. Where acid neutralization is practiced, the agent should be applied at full strength (100% concentration), or at a minimum of a 50/50 mix with water.

A light cleaner suitable for home use, light cleaning operations (such as a car wash), clearing blocked or partially blocked drains (including septic tanks), and as a dishwashing additive, can also be produced. The light cleaner has the same components as the industrial cleaner set forth above except for the composition of the detergent mixture. For light cleaning functions, the detergent mixture composition would be (by volume): about 18-28% LAS, about 5-8% IP, about 37% LD, about 0.25-0.75% MS, and about 56.25-73.75% water. This light cleaner will not cause any stains on carpet, damage finishes of paint work or the like and may easily be taken up after cleaning. It is safe to store and handle with no special warning labels being necessary, it is nontoxic, non-flammable, and safe in all household and light industrial drainage systems including those with septic tanks.

Deodorant

According to yet another aspect of the present invention, a formulation is provided which may be used as a commercial deodorant either in concentrated form, or mixed with water. The fragrance utilized in the deodorant may be changed to order, and fragrances will be properly held in place and the mixture will remain intact and will not separate. The deodorant is non-toxic, biodegradable, has a low pH, is non-flammable and non-combustible, will not harm the environment, and may be used as a solid, liquid, or particlized spray. The deodorant may be used in all heavy industrial and farm uses where odors are a problem, and all light industrial areas where odors are a problem, and in home environments, and may be added to conventional liquid cleaners to produce a clean and fresh smell.

A composition of the preferred deodorizer according to the invention is as follows: about 5-50% by volume of the deodorizer of detergent mixture; about 0.25-1.5% (by weight of the detergent mixture) vitamin B-6; about 1-15% (by weight of detergenh mixture) sodium bicarbonate; about 0.001-6% (by weight of the detergent mix) of a fragrance, such as eucalyptus oil; and water in an amount of 26.5-93.75% by volume of the total deodorizer. Vegetable color may also be added, in an amount of about .001-1% by weight of the detergent mixture. The preferred composition of the detergent mixture is (by volume): about 5-60% LAS, about 0.25-20% IP, about 0.25-12% LD, about 0.1-1% MS, and about 7-94.49% water. The bicarbonate of soda, and the LAS are the primary vehicles that give the deodorizer viscosity to hold the fragrance. Even when concentrated deodorizer is diluted, the mixture remains intact and will not separate. At higher concentration (minimum amount of water in both the detergent mixture and the deodorizer composition in general) it may be a semi solid that can be remixed and cut to any consistency desired. At the lower concentrations it is immediately available for use. For the heavy concentrate the amount added to water or conventional cleaners is typically about 0.5 oz per gallon for most applications. When it is used as a self contained spray, it will typically be diluted with 100 parts water to one part concentrate. In areas where the odors are particularly offensive, such as animal manure pits, heavier concentration deodorizer should be utilized, and should be mixed as three parts concentrate to 97 parts water.

Various formulation according to the present invention may also be put to other uses. For instance the oil dispersant also can be useful in explosion prevention since it can effect high speed emulsification of fuel vapors (including air craft explosion prevention, deep mine methane explosion prevention, and boiling liquid expanding vapor explosion prevention). It also can be used for dust explosion prevention, such as coal dust or grain elevator dust. The class A fire fighting agent also can be used as a fire and explosion preventing substance at oil well drilling sites, since it will not froth with heavy oil fires as existing foams have a tendency to do. The class B and D fire fighting agent according to the invention also may be used as a liquid insulating agent. Further, a number of the formulations according to the invention can be used as fire retarders for different building products, such as wood and wood product building products.

It will be thus be seen that according to the present invention a liquid formulation having excellent effectiveness for a wide variety of uses has been provided.

**Claims**

1. A liquid formulation for use as a Class D firefighting agent and comprising a mixture of:

a linear alkylbenzene sulfonate, non-ionic detergent and lauric superamide detergent mixture comprising about 39-67 percent of the total mass of the formulation;

vitamin B-6 in the amount of 1-3 percent by weight of the detergent mixture;

sodium chloride in the amount of about 3-41 percent by weight of the detergent mixture;

bicarbonate of soda in the amount of about 3-20 percent by weight of the detergent mixture;

0-4 percent by weight of the detergent mixture coloring and perfuming agents; and

a volume of water large enough only to provide effective mixing of the other components of the formulation and insufficiently large to interfere with the use of the formulation as an effective Class D firefighting agent.

2. A method of extinguishing a combustible metal fire comprising the steps of:

applying directly to the fire a liquid composition comprising a major part of detergent mixture of linear alkylbenzene sulfonate, non-ionic detergent and lauric superamide, and amounts of vitamin B-6, sodium chloride, bicarbonate of sodium, with minimal amounts of water, and with small amounts of coloring and perfuming agents, if desired, effective when, in formulation, to put out a combustible metal fire, by slowing down the detergent mixture emulsification rate; densifying and increasing the heat absorption capability of the detergent mixture; agitating and stabilizing the detergent mixture; and densifying and preventing separation and detonation of the detergent mixture when exposed to burning metal.

3. A formulation as recited in claim 1 wherein the detergent mixture component of the formulation comprises, by volume about 41-45% linear alkylbenzene sulfonate, about 8-12% isooctylphenyl polyethoxyethanol, about 0-4 polyoxyethylene sorbitan monooleate, about 8-12% lauric diethanolamide, about 0.5-1% monoethanolamide superamides, and about 26-30% water.

4. A formulation for use in extinguishing coal fires, without generation of substantial gases toxic to humans, for metering to the fire at about a 6-10 percent dilution rate to water, the formulation comprising a mixture of:

a linear alkylbenzene sulfonate, non-ionic detergent and lauric superamide detergent mixture comprising about 50 percent by volume of the formulation;

vitamin B-6 in the amount of about 0.5-3 percent by weight of the detergent mixture;

bicarbonate of soda in the amount of about 3-18 percent by weight of the detergent mixture; and

water comprising about 37-47 percent by volume of the total formulation.

5. A method of extinguishing a coal fire comprising the step of:

applying directly to the burning coal a liquid composition containing a detergent mixture of linear alkylbenzene sulfonate, non-ionic detergent and lauric superamide and water, with effective amounts of materials for densifying and increasing the heat absorption capability of the detergent mixture and water; and slowing down the emulsification rate of the detergent mixture, so as to render the liquid composition effective for extinguishing a coal fire, and without generation of gases toxic to humans.

6. A method of fighting a coal fueled fire comprising the steps of:

(a) applying directly to the burning coal a liquid composition containing a mixture of: a linear alkylbenzene sulfonate, non-ionic detergent and lauric superamide detergent mixture; vitamin B-6; bicarbonate of soda; water; and red dye so as to put out the coal fueled fire without generation of gases toxic to humans;

(b) directing a light on portions of the coal to which the liquid composition has been applied;

(c) detecting the reflection of the light off of the coal to which the liquid composition has been applied; and

(d) proceeding past the area upon which the light has been directed once there is a reflection of red colored light, provided by the red dye, from that area.

7. A formulation comprising a linear alkylbenzene sulfonate, non-ionic detergent and lauric superamide detergent mixture; water; vitamin B-6; and bicarbonate of soda; and lemon or lime juice.

8. A formulation as recited in claim 7 for use as an oil dispersant for application at about a 25% dilution rate to water, and wherein: the detergent mixture comprises about 50% of the formulation total volume, vitamin B-6 comprises about 3-10% of the weight of the total formulation, bicarbonate of soda comprises about 1-7% by weight of the formulation, lemon or lime juice comprises about 1-3% by weight of the formulation, water comprises about 26-44% of the volume of the formulation, and less than 4% by weight of the formulation comprises non-toxic coloring and/or perfuming agents.

9. A formulation for use as a Class B firefighting agent, for application with water at a dilution rate of about 0.5-6 percent water, the mixture comprising:

a linear alkylbenzene sulfonate, non-ionic detergent and lauric superamide detergent mixture comprising about 39-67 percent of the total mass of the formulation;

vitamin B-6 in the amount of about 0.5-3 percent by weight of the detergent mixture;

sodium chloride in the amount of about 3-33 percent by weight of the detergent mixture; and

bicarbonate of soda comprising about 1-20 percent by weight of the detergent mixture.

10. A method of extinguishing a Class A or a Class B fire comprising the steps of:

mixing with water a liquid composition comprising about 22-45 percent by volume of the composition a detergent mixture of alkylbezene sulfonate, non-ionic detergent and lauric superamide; alfalfa in an amount of about 0-10 percent by weight of the detergent mixture; vitamin B-6 in an amount of 0.5-1.5 percent by weight of the detergent mixture; bicarbonate of soda in an amount of about 0.25-20 percent by weight of the detergent mixture; and about 50 percent by volume water;

diluting the liquid composition with water at a dilution rate of about 3-6 percent; and

spraying the liquid composition diluted with water directly onto a Class A or Class B fire.

11. A formulation comprising:

about 45-75% by volume of a linear alkylbenzene sulfonate, non-ionic detergent and lauric superamide detergent mixture; about 0.5-3% by weight of the detergent mixture of vitamin B-6; and about 25-55% by volume water, the formulation having a pH between about 7.6-8.

12. A method of deodorizing an environment with offensive odors comprising the steps of:

(a) producing a formulation comprising: linear alkylate sulfonate, non-ionic detergent and lauric superamide mixture, comprising about 5-50 by volume of the formulation; vitamin B-6 in an amount of about 0.25-1.5% by weight of the detergent mixture; sodium bicarbonate in an amount of about 1-15% by weight of the detergent mixture; a fragrance in the amount of about 0.001-6% by weight of the detergent mixture; and water comprising 26.5-93.75% by volume of the formulation; and

(b) applying the formulation directly to the environment with offensive odors, to effect deodorization.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | US-A-4 725 370 (J.S. GREENE)(16-02-1988) * Whole document * | 4-6 | A 62 D 1/00 |
| X | US-A-4 636 325 (J.S. GREENE) * Claims 1-6 * | 4 | C 11 D 1/86 |
| A | | 1,3,4,7 -9,11 | C 11 D 3/28 |
| D,A | US-A-4 248 733 (J.B. STATES Sr.) * Whole document * | 1-11 | |
| A | GB-A-1 516 977 (PROCTOR & GAMBLE) * Claims * | 1,3,4,7 -9,11 | |

**TECHNICAL FIELDS SEARCHED (Int. Cl.4)**

A 62 D
C 11 D

The present search report has been drawn up for all claims

| Place of search | Date of completion of the search | Examiner |
|---|---|---|
| THE HAGUE | 02-05-1988 | FLETCHER A.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)